(19) Europäisches Patentamt
European Patent Office
Office européen des brevets

(11) Publication number: **0 209 363 B1**

(12) **EUROPEAN PATENT SPECIFICATION**

(45) Date of publication of patent specification: **06.05.92**

(51) Int. Cl.$^5$: **C07K 5/06**, C12P 37/00, //C12R1/75,C12R1/82, C12R1/465

(21) Application number: **86305447.4**

(22) Date of filing: **15.07.86**

(54) **Heteropeptides, intermediates in the biosynthesis of penicillins.**

(30) Priority: **15.07.85 ES 545211**
**15.07.85 ES 545213**
**07.11.85 ES 548662**
**07.11.85 ES 548675**
**07.11.85 ES 548676**

(43) Date of publication of application:
**21.01.87 Bulletin 87/04**

(45) Publication of the grant of the patent:
**06.05.92 Bulletin 92/19**

(84) Designated Contracting States:
**AT BE CH DE FR GB IT LI LU NL SE**

(56) References cited:

**CHEMICAL ABSTRACTS, vol. 106, no. 11, 16th March 1987, page 477, abstract no. 83040u, Columbus, Ohio, US; & ES-A-545 213 (ANTIBIOTICOS S.A.) 16-02-1986**

(73) Proprietor: **Antibioticos, S.A.**
**Bravo Murillo 38**
**28015 Madrid(ES)**

(72) Inventor: **Luengo, Jose Maria c/o Antibioticos S.A.**
**Fabrica Avda De Antibioticos 59-61**
**Apartada Num 255 Leon(ES)**
Inventor: **Lopez-Nieto, M. J. c/o Antibioticos S.A.**
**Fabrica Avda De Antibioticos 59-61**
**Apartada Num 255 Leon(ES)**
Inventor: **Salto, Francisco c/o Antibioticos S.A.**
**Fabrica Avda De Antibioticos 59-61**
**Apartada Num 255 Leon(ES)**

(74) Representative: **Ruffles, Graham Keith et al**
**MARKS & CLERK 57-60 Lincoln's Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 209 363 B1

Rank Xerox (UK) Business Services
(3.08/2.18/2.0)

CHEMICAL ABSTRACTS, vol. 107, no. 9, 31st August 1987, page 563, abstract no. 76081a, Columbus, Ohio, US; & ES-A-548 662 (ANTIBIOTICOS S.A.) 16-03-1986

CHEMICAL ABSTRACTS, vol. 107, no. 9, 31st August 1987, page 563, abstract no. 76082b, Columbus, Ohio, US; & ES-A-548 676 (ANTIBIOTICOS S.A.) 16-03-1986

CHEMICAL ABSTRACTS, vol. 104, no. 21, 26th May 1986, page 509, abstract no. 184806n, Columbus, Ohio, US; J.M. LUENGO et al.: "Direct enzymic synthesis of penicillin G using cyclases of Penicillium chrysogenum and Acremonium chrysogenum", & BIO/TECHNOLOGY 1986, 4(1), 44-7

CHEMICAL ABSTRACTS, vol. 105, no. 7, 18th August 1986, page 275, abstract no. 56843f, Columbus, Ohio, US; M.J. CASTRO et al.: "Conversion of phenylacetyl-cysteinyl-valine in vitro into penicillin G by isopenicillin N synthase of Streptomyces lactamdurans", & FEMS MICROBIOL. LETT. 1986, 34(3), 349-53

THE JOURNAL OF ANTIBIOTICS, vol. 39, no. 6, June 1986, pages 822-826; S.E. JENSEN et al.: "Synthesis of benzylpenicillin by cell-free extracts from Streptomyces clavuligerus"

JOURNAL OF THE CHEMICAL SOCIETY, CHEMICAL COMMUNICATIONS, no. 24, 15th December 1985, pages 1808,1809, The Royal Society of Chemistry; J.E. BALDWIN et al.: "Penicillin biosynthesis: direct biosynthetic formation of penicillin V and penicillin G"

## Description

### BACKGROUND OF THE INVENTION

This invention relates to penicillins.

The tripeptide δ-(L-α-aminoadipyl)-L-cysteinyl-D-valine ("ACV") is a key intermediate in the biosynthesis of β-lactam antibiotics such as penicillins, cephalosporins and cephamycins. To be more specific, it is apparent that Penicillium chrysogenum and Acremonium chrysogenum (also known as Cephalosporium acremonium) share initial steps in the biosynthesis of β-lactams. In the biosynthesis, the tripeptide ACV is formed from L-α-aminoadipate, L-cysteine, and L-valine, followed by cyclization of ACV to isopenicillin N. Thereafter, the biosynthetic pathways diverge. With Penicillium, the L-α-aminoadipyl side chain is exchanged for a hydrophobic one, for example, a phenylacetyl side chain in the case of penicillin G (benzylpenicillin). With Acremonium, the L-α-aminoadipyl side chain is epimerized to the D-form, giving penicillin N, followed by expansion of the thiazolidine ring of the penicillin N to give deacetoxycephalosporin C.

Studies have been carried out by various research teams on the substrate specificity of the cyclization enzyme (isopenicillin N synthetase, also called cyclase) which acts to cyclize ACV to isopenicillin N. It has been established that the isopenicillin N synthetases of P. chrysogenum and A. chrysogenum show a narrow substrate specificity. For example, it has been demonstrated that a tripeptide containing glycine in the place of valine will block the cyclization process. It has also been shown that ACV cyclization is inhibited when the related tripeptide glutathione (γ-glutamyl-cysteinyl-glycine) is added to the reaction mixture.

In general, the results show that there is some flexibility in the nature of the amino acid (that is, D-valine) at the third position of the tripeptide, no flexibility in the second position (L-cysteine), and only limited flexibility in the first position (L-α-aminoadipic acid). Thus, although L-carboxymethylcysteine, adipic acid or D-α-aminoadipic acid can replace L-α-aminoadipic acid at the first position of the tripeptide, there are many more acids which can not, including L-glutamic acid, L-aspartic acid, caproic acid, delta-aminovaleric acid, glutaric acid, aminopimelic acid, and acetic acid. Indeed, the Oxford team of Baldwin et al. (J. Chem. Soc. Chem. Commun, 1225-1227, 1984) recently concluded that the first residue of the tripeptide must have a six-carbon or equivalent chain terminating in a carboxy group, in order for successful conversion to a penicillin.

Furthermore, it is thought that the failure of a number of attempts to force Cephalosporium to produce directly a phenylacetylcephalosporin by feeding phenylacetic acid arises presumably because Cephalosporium lacks the penicillin acyltransferase enzyme thought to be necessary.

### OBJECTS OF THE INVENTION

It is an object of this invention to provide new routes to penicillins and related compounds. A further object is the provision of new substrates for the enzyme cyclase.

### SUMMARY OF THE INVENTION

In an unexpected manner, it has been found that a cyclase can convert the heteropeptide phenylacetyl-L-cysteinyl-D-valine ("PCV") directly in to benzylpenicillin (penicillin G) and the heteropeptide phenoxyacetyl-L-cysteinyl-D-valine ("POCV") to penicillin V, and can analogously convert other arylacyl-L-cysteinyl-D-valine heteropeptides to penicillins.

Thus, the present invention provides a process for producing a penicillin which comprising cyclizing PCV, POCV or other PCV analogue, using a cyclase.

The heteropeptides PCV and POCV are themselves novel compounds. The formulae of these novel compounds are as follows:

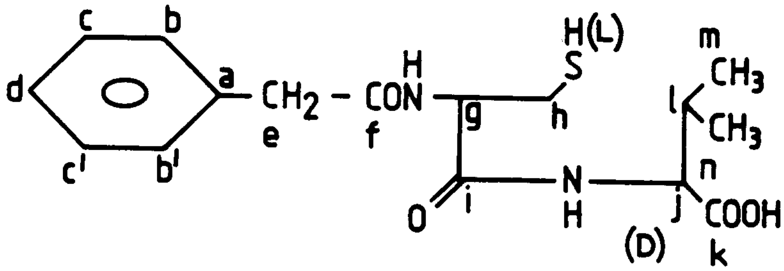

PCV

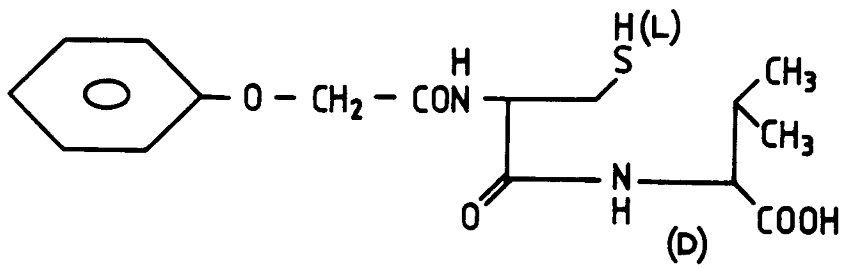

POCV

More generally, the heteropeptides for use in the present invention are novel compounds, and form part of this invention.

DESCRIPTION OF THE PREFERRED EMBODIMENTS

The present invention is based on the surprising discovery that the heteropeptides PCV and POCV can be directly converted to benzylpenicillin and penicillin V respectively by cell-free extracts of Acremonium chrysogenum. Further work has indicated that other enzyme sources can be used. The yields are currently relatively low, but nevertheless the extent of conversion is significant and does clearly indicate the affinity of the cyclase for the arylacyl heteropeptides.

The cyclase for use in the process of this invention is typically employed as a cell-free extract of a cyclase-producing micro-organism of the genus Cephalosporium or Penicillium (for PCV or POCV) or Streptomyces for (PCV only). Suitable species include C. acremonium (=A. chrysogenum), P. chrysogenum and (for use with PCV only) Streptomyces clavuligerus. Cell-free extracts can be obtained by harvesting the culture, resuspending the cells in a suitable buffered medium, distrupting the cells for example by sonification and then centrifuging to generate the crude extract. The crude cyclase may be purified by conventional techniques.

As an alternative to cell-free extracts, it is possible that dead, lysed cells or cells rendered permeable to the heteropeptide might be employed: a simple test will determine if a cyclase sources is converting the heteropeptide substrate to the desired β-lactam.

Immobilized forms of the cyclase or cyclase-containing material can be employed, using conventional immobilization techniques.

The heteropeptides are conveniently prepared and stored as disulphide dimers arising from the presence of the thio group in the cystine. The bis-heteropeptide may then be converted to the mono form in situ by the use of an appropriate reducing agent, for example dithiothreitol.

For small scale investigative work, suitable conditions might involve incubating cell free extracts of the cyclase (5 to 20 mg of protein /ml) at a 20 to 37°C for 0 to 120 minutes in a suitable buffered reaction mixture, such as the following reaction mixture: 30mM Tris-HCl buffer pH 7.5 to 8.1, 4 to 6mM DTT, 0.1 to 0.5mM $FeSO_4$, 1 to 3mM Na ascorbate and 0.2-3mM bis-heteropeptide. The reaction can be stopped by the addition of methanol.

The formation of penicillin may be assayed using a bioassay technique, for example by plating the sample on to agar inoculated with a susceptible bacterium. The reaction products can readily be identified by HPLC and other chromatographic techniques known in the art.

Furthermore, the penicillins can be prepared as salts or esters, for example by salification or esterification of a free acid.

The isolated penicillin can be formulated in conventional manner with pharmaceutical carriers in order to provide suitable dosage forms.

## THE DRAWING

In the following Examples, reference is made to the accompanying drawing, in which:

Figure 1 is a graph with time of benzylpenicillin synthesis and isopenicillin N synthesis, respectivley from PCV and ACV, using enzyme extracted from Streptomyces clavuligerus.

Figure 2 shows HPLC traces of (a) pure benzylpenicillin, (b) the reaction product of PCV cyclization using P. chrysogenum cyclase and (c) the reaction product of PCV cyclization treated with penicillin acylase.

## EXAMPLES OF THE INVENTION

For the Examples, penicillin N (95% purity by weight), potasssium salt of benzylpenicillin (1580 U/mg), penicillin acylase from E. coli and (L-α-amino-δ-adipyl)-L-cysteinyl-D-valine (ACV) were supplied by Antibioticos S.A., Leon, Spain. Dithiothreitol (DTT), Tris, protamine sulphate and bovine serum albumin were obtained from Sigma Chem. Co. (St. Louis. U.S.A.). Other chemicals used were reagent quality or HPLC grade. Streptomyces clavuligerus ATTC 27054 and Micrococcus luteus ATCC 9341 were obtained from the American Type Culture Collection.

## EXAMPLE 1

### SYNTHESIS OF PHENYLACETYL-L-CYSTEINYL-D-VALINE (PCV)

Phenylacetyl-L-cysteinyl-D-valine (PCV) was synthesized from phenylacetyl-S-trityl-L-cysteine (previously obtained by reaction of phenylacetyl chloride with S-trityl-L-cysteine) and p-toluenesulphonate-D-valine-benzyhydryl ester using the N-ethoxycarbonyl-2-ethoxy-1,2-dihydroquinoleine technique (EEDQ) described in J. Amer. Chem. Soc. 90:1651-1652. Protected PCV was synthesized by this procedure, and the dimer bis-PCV was finally obtained by hydrolysis. The PCV was used as the free thiol, or the disulphide dimer was reduced to the monomer in situ with dithiothreitol (DTT).

Samples of bis-PCV were hydrolyzed in a vacuum-sealed tube with 5.9 M tridistilled HCl containing 0.1% (v/v) phenol for 25 hours to determine the PCV composition. After this treatment, samples were dried in a desiccator with NaOH, washed twice with bidistilled water and analyzed with a Durrum D-500 amino acid autoanalyzer. A sample of cystine was treated in identical conditions at the same time and no destruction of this molecule was observed. Cystine and valine were present in the correct proportions. The remaining product was identified by gas chromatography as phenylacetic acid using a Hewlett-Packard gas chromatograph with a column of diethyleneglycol-succinate (20%) at 170°C.

PCV was also synthesized according to the methodology described by Wolfe and Jokinen in Can. J. Chem. 57:1388-1396 1979, using phenylacetic acid instead of L-α-aminoadipic acid.

Physical properties of the PCV were as follows:
Melting point;
190 ° C (dec.).
Optical Rotation; $[\alpha]_D^{25}$
-73.05 (phosphate buffer 50 mM, pH 8.0).
Infrared Spectroscopy; (KBr) $cm^{-1}$
3400 (N-H)
3300 (br, acid O-H)
1750 (amide, C = O)
1670 and 1550 (phenyl group)
1355 and 1400 [$(CH_3)_2$-CH].
Ultravoilet Spectrometry; $\lambda_{max}$
210
250
256
264.
Nuclear Magnetic Resonance;
$^1H$ NMR (80MHz, $CD_3OD$) $\delta$
0.85 and 0.90 (Hm*, Hn, 2d, 6.8 Hz),
2.07 (Hl, m),
3.06 (Hg, Hh, m),
3.57 (He, s),
4.28 (Hj, d, 5.4 Hz),
7.27 (H$\phi$, s).

$^{13}C$ NMR (20,lMz, $D_2O$, pH 9.0 with NaOH) $\delta$
175.43 or 175.48 (Ci**, Ck, 2s),
172.9 (Cj, s),
130.01 or 130.22 (Cc, Cc', Ca, dd, s),
128.18 or 128.40 (Cd, Cb, Cb', 3d),
61.67 (Cj, d),
59.84 (Cg, d),
43.23 or 43.30 (Ce, Ch, 2t),
31.79 (Cl, d),
18.18 or 19.99 (Cm, Cn, 2q).

## EXAMPLE 2

### PREPARATION OF ISOPENICILLIN N SYNTHETASE FROM STREPTOMYCES CLAVULIGERUS AND DETERMINATION OF CYCLIZATION ACTIVITY AND REACTION PRODUCTS USING PCV AND ACV AS SUBSTRATES.

*S. clavuligerus* ATTC 27064 was maintained and grown as described by O'Sullivan *et al.* in Biochem. J. 179:47-52, 1979. 250 ml of culture were harvested by centrifugation at 10,000 x g for 5 minutes, washed four times by resuspension in sterile saline solution and centrifuged as before. The precipitated cells, resuspended in 25 ml of 50 ml Tris-HCl buffer pH 7.5 containing 0.1mM DTT (TD buffer), were sonicated as described in J. Antibiot. 35:483-490, 1982, using a Branson Sonifier Cell Disruptor (B-12). The broken cell suspension was ultra-centrifuged at 100,000 x g (Beckman L5-65B) for 90 minutes, treated with 0.2 (w/v) protamine sulphate and the clear extract precipitated with ammonium sulphate (30-80% saturation, for example 50-80% saturation). The resulting precipitate was resuspended in 3 ml of TD buffer and applied to

*the positions of the hydrogen atoms are indicated with reference to the letters shown on the formula given above for PCV.

**the positions of the carbon atoms are indicated with reference to the letters shown on the formula given above for PCV.

a Sephadex (Trade Mark) G-25, PD-10 column (Pharmacia). Aliquots of 1 ml were collected and assayed for isopenicillin N synthetase (cyclase) activity. Fractions 4 and 5, containing maximal activity, were mixed and employed for the reaction. The cyclase extracts were stored frozen at -20 C. Protein was measured by the method of Lowry et al described in J. Biol. Chem. 193:265-273, 1951.

Cyclization activity of the cyclase on ACV or PCV was assayed by a slightly modified version of the method described by Jensen et al. in J.Antibiot, 35:483-490, 1982 and in Antibiotics and Other Secondary Metabolites pages 141-164, Academic Press. The reaction mixture contained 30mM Tris-HCl buffer pH 7.5; 0.5 mM $FeSO_4$; 1mM bis-ACV or 3mM bis-PCV; 4mM DTT; 3 mM Na ascorbate and enzyme (10mg of protein) in a final reaction volume of 0.5 ml. The reaction mixture was incubated at 25° C for 90 min and stopped by adding 0.5ml of methanol.

In various experiments it was shown that the cyclization of the tripeptide ACV by isopenicillin N synthetase extracted from S. clavuligerus generated isopenicillin N, whereas the cyclization of PCV resulted in benzylpenicillin.

The formation of isopenicillin N and benzylpenicillin was measured by bioassay against M. luteus ATCC 9341 following the hole plate method described in Biochem. J. 103:877-890, 1967. Penicillin N and benzylpenicillin were used as standards. One unit of antibiotic (penicillin N or benzylpenicillin) gave a zone of inhibition equivalent to 1μg or 1.58 μg of penicillin N or benzylpenicillin respectively with M. luteus luteus ATCC 9341 as test organism.

Cyclization of PCV led to the formation of a molecule active against M. luteus ATTC 9341 and sensitive to β-lactamase I from Bacillus careus (Difco) (Table 1).

The cycllization product of PCV lost over 90 per cent of its antibacterial activity when incubated with 2 I.U. of penicillin acylase at 30° C for 10 minutes. In contrast the isopenicillin N obtained from the cyclization of ACV was not sensitive to penicillin acylase (Table I ).

Table 1.

| Conversion of peptides to penicillin by S. clavuligerus cyclase. | | |
|---|---|---|
| Substrate | Reaction mixture | Isopenicillin N[1] or Benzylpenicillin[2] (U/ml) |
| ACV | Control<br>+ Penicillin acylase (2 I.U, 10 min.)<br>+ β-lactamase | 40<br>38<br>0 |
| PCV | Control<br>+ Penicillin acylase (2 I.U, 10 min.)<br>+ β-lactamase | 0.15<br>0.01<br>0.00 |
| 1 Isopenicillin N was measured using penicillin N as standard<br>2 Benzylpenicillin was measured using benzylpenicillin as standard. | | |

Controls performed individually without substrate (PCV) and without enzyme in the reaction mixture were negative. These results are shown in Table 2.

Table 2

| | Benzylpenicillin (U/ml) |
|---|---|
| Complete reaction mixture | 0.15 |
| Without PCV | 0.0 |
| Without enzyme | 0.0 |

The kinetics of cyclization with the cyclase were studied. Fig 1 shows that formation of penicillin G from PCV followed a slow time dependent course whereas synthesis of isopenicillin N from ACV was much faster. Under the above cyclization conditions, 0.15 U/ml of benzylpenicillin were produced, while in similar conditions, 40 U/ml of isopenicillin N (evaluated as penicillin N) were obtained.

Isopenicillin N from the reaction mixture was identified under the conditions described by Jensen et al in J. Antibiot. 35:1026-1032, 1982. Benzylpenicillin obtained by cyclization of PCV, was identified by the following method. Five reactions (200 ml) were carried out using PCV as substrate in order to isolate sufficient reaction product for identification. After incubation, each reaction mixture was vigorously extracted with 30 ml of chilled amyl acetate containing 2.2 ml of phosphoric acid (83% v/v, density 1.7) according to the method described by Revilla et al in J. Antibiotic. 37:781-789. The extracted product was transferred to 10ml of 50mM phosphate buffer pH 7.0 and re-extracted with 3ml of amyl acetate containing 10µl of phosphoric acid. The product in the organic phase was transferred again to 1 ml of 50mM phospate buffer, pH 7.0 and kept in a refrigerator. The five processed samples were mixed, re-extracted with 2 ml of amyl acetate containing 8 µl of phosphoric acid and transferred to 750 µl of phosphate buffer. By this procedure about 100 µg of benzylpenicillin were obtained. Purification of the penicillin was carried out by HPLC using a Perkin Elmer Chromatograph Series 3B with a LC-7 spectrophotometric detector and a Sigma 10 B Chromatography Data Station. A µ-Bondapack C18 column of 30cm (Waters Associates Inc.) and a mobile phase composed of 83% (v/v) of 0.1 M sodium acetate/acetic acid pH 4.5 and 17% (v/v) of acetonitrile was used. The retention time of benzylpenicillin under these conditions was 12.3 minutes.

Benzylpenicillin was also identified by descending paper chromatography (Whatman No.1 paper buffered with 10% sodium citrate at pH 3.7) using the following solvent systems 1) pyridine:n-butanol:water (1:1:1) II) n-butanol:ethanol:water (4:1:5, upper phase); by thin layer electrphoresis in pyridine: acetic acid: water (1:10:135) pH 3.5, 300 v/cm for 2 hours; and by paper electrophoresis at 13 v/cm in collidine: acetate buffer (0.05 M acetate) pH 7.0 for 3 hours. The spots of penicillin and its transformation product penicilloic acid (obtained by incubation with β-lactamase from B. cereus $UL_1$ were located by spraying the paper or thin layer with 0.5 M NaOH followed by starch iodine as described by Lopez-Nieto page 77 of phD Thesis, University of Salamanca, Spain.

## EXAMPLE 3

### PREPARATION OF ISOPENICILLIN N SYNTHETASE FROM PENICILLIUM CHRYSOGENUM AND DETERMINATION OF CYCLIZATION ACTIVITY AND REACTION PRODUCTS USING PCV AND ACV AS SUBSTRATES.

Example 2 was repeated but using isopenicillin N synthetase extracted from a high producing strain of Penicillium chrysogenum (Penicillium chrysogenum AS-P-78, Luengo et al J. Antibiot. 144:869-876).

Cells containing high isopenicillin N synthetase activity were obtained in a two stage process as described in J. Antibiot. 144:869-876. Cell free extracts were obtained as described by Ramos et al in Antimicrob. Agents Chemother. 27(3):380-387. Mycelium of P. chrysogenum was collected at 24 hours of cultivation in cephalosporin production medium (CP medium) and resuspended in 30mM Tris-HCl buffer pH 8.0 containing 0.1mM dithiothreitol (DTT). The mycelium was disrupted by sonification for 5 minutes. The extract was centrifuged at 100,000 x g for 1 hour at 4°C in a Beckman L8-70 ultracentrifuge and the resulting crude extract was stored at -20°C. Isopenicillin N synthetase were purified as previously described in Antimicrob. Agents Chemother. 27(3):380-387.

As in Example 2, the use of the extracted enzyme resulted in the cyclization of ACV to isopenicillin N and PCV to benzylpenicillin. Both reaction products were completely inactivated by penicillinase. When PCV was used as the substrate, the reaction product was inactivated by penicillin acylase. Inactivation of the reaction product by penicillin acylase did not occur with ACV as substrate.

The isopenicillin N and benzylpenicillin formed by cyclization of 0.2mM ACV or 2mM PCV using cyclase of A. chrysogenum was assayed by the following bioassay. After the reactions were stopped with methanol, triplicate 50 µl samples were applied to wells 7mm in diameter in plates containing 15ml of Tryptic Soy Agar (TSA) medium (Difco) innoculated with Micrococcus luteus (0.5 ml of a culture having an O.D. of 1.0 at 600nm). Control samples of pure isopenicillin N or benzylpenicillin were treated with methanol in a similar way to the reaction samples. The samples in the assay plates were kept at 4°C for 3 hours to allow diffusion and then incubated at 30°C for 12 hours.

Figure 2 shows HPLC elution patterns of (a) pure benzylpenicillin (retention time 12.3 minutes); (b) the reaction product of PCV cyclization using P. chrysogenum cyclase after solvent extraction and (c) the reaction product of PCV cyclization treated with penicillin acylase and extracted with solvent. Conditions were as given previously for isolation and charaterization of the cyclization products.

The location after chromatography of the antibiotic substance obtained by cyclization of PCV with extracts of P. chrysogenum was also established by cutting the chromatography paper into 1.5 x 0.5 cm segments and testing each piece on a plate of TSA previously seeded with a lawn of E. coli ESS or M.

luteus. The activity against both microorganisms was located in the segment having an Rf of 0.67 (benzylpenicillin). When similar experiments were carried out with samples in which ACV was the substrate of the cyclase, the bioactivity was located in the first and second segment (Rf 0.06).

## EXAMPLE 4

### PREPARATION OF ISOPENICILLIN N SYNTHETASE FROM ACREMONIUM CHRYSOGENUM AND DETERMINATION OF CYCLIZATION ACTIVITY AND REACTION PRODUCTS USING PCV AND ACV AS SUBSTRATES.

Example 2 was repeated but using isopenicillin N synthetase extracted from a high producing strain of Acremonium chrysogenum (A. chrysogenum C-10 available from A. L. Demain).

Cells containing high isopenicillin N synthetase activity were obtained in a two stage process as described in J. Antibiot. 144:869-876. A. chrysogenum cultures were developed in seed medium for 4 days and these used to innoculate 60ml of chemically defined cephalosporin production medium, (CP medium). Cell free extracts were obtained as described by Ramos et al in Antimicrob. Agents Chemother. 27(3):380-387. Mycelium of A. chrysogenum was collected at 64 hours of cultivation in CP medium and resuspended in 30mM Tris-HCl buffer pH 8.0 containing 0.1mM dithiothreitol (DTT). The mycelium was disrupted by sonification for 2 minutes. The extract was centrifuged at 100,000 x g for 1 hour at 4° C in a Beckman L8-70 ultracentrifuge and the resulting crude extract was stored at -20 C. Isopenicillin N synthetase was purified as previously described in Antimicrob. Agents Chemother. 27(3):380-387.

As in Examples 2 and 3, the extracted enzyme resulted in the cyclization of ACV to isopenicillin N and PCV to benzylpenicillin. Both reaction products were completely inactivated by penicillinase but only when PCV was used as the substrate was the reaction product inactivated by penicillin acylase.

Bioassay of the isopenicillin N and benzylpenicillin formed by the A. chrysogenum cyclase was performed in a similar way to the method described in Example 3 for assaying the penicillins produced by the cyclase extracted from P. chrysogenum.

The location after chromatography of the antibiotic substance obtained by cyclization of PCV with extracts of A. chrysogenum was established as in Example 3 and similar results obtained.

## EXAMPLE 5

### PREPARATION OF ISOPENICILLIN N SYNTHETASE FROM PENICILLIUM CHRYSOGENUM AND DETERMINATION OF CYCLIZATION ACTIVITY AND REACTION PRODUCTS USING POCV AS SUBSTRATE.

Example 3 was repeated but using phenoxyacetyl-L-cysteinyl-D-valine (POCV) as substrate in place of PCV.

POCV was prepared by either standard methods described by Baldwin et al in J. Chem Soc. Perkin. Trans. 1,1981,2253 or by N-acylation of cystine, followed by ethyl 1,2-dihydro-2-ethoxy-1-quinoline carboxylate (EEDQ) coupling with valine benzhydryl ester and deprotection with triflouroacetic (TFA) to give the heteropeptide as the disulphide.

The cell free extracts of P. chrysogenum AS-P-78 (5-20 mg of protein/ml) were incubated at 25° C for 30 minutes in a reaction mixture containing 30mM Tris-HCl buffer pH 8.1, 0.1mM $FeSO_4$, 6mM DTT, 3mM Na ascorbate and 3mM POCV. The pH was adjusted to 2.0.

The cyclization of POCV by isopenicillin N synthetase extracted from P. chrysogenum generated penicillin V. Controls performed individually without subtrate (POCV) and without enzyme were negative. The results are given in Table 3 below.

Table 3

| | Penicillin V (U/ml) |
|---|---|
| Complete reaction mixture | 0.18 |
| Without PCV | 0.0 |
| Without enzyme | 0.0 |

The reaction product was identified as penicillin V by HPLC and other chromatography techniques according to the methods described in Example 2.

EXAMPLE 6

PREPARATION OF ISOPENICILLIN N SYNTHETASE FROM ACREMONIUM CHRYSOGENUM AND DETERMINATION OF CYCLIZATION ACTIVITY AND REACTION PRODUCTS USING POCV AS SUBSTRATE.

Example 4 was repeated but using POCV as substrate in place of PCV. POCV was prepared as described in Example 5. The cyclase was extracted from A. chrysogenum AS-C-80 and incubated in the reaction mixture described in Example 5, with the exception that the pH of the Tris buffer was 7.8.

The results of the incubation of the cyclase with POCV are shown in Table 4.

Table 4

| | Penicillin V (U/ml) |
|---|---|
| Complete reaction mixture | 0.25 |
| Without PCV | 0.0 |
| Without enzyme | 0.0 |

The reaction product was identified as penicillin V by HPLC and other chromatography techniques according to the methods described in Example 2.

**Claims**

1. An arylacyl-cysteinyl-valine heteropeptide selected from phenylacetyl-L-cycsteinyl-D-valine ("PCV") and phenoxyacetyl-L-cysteinyl-D-valine ("POVC").

2. The heteropeptide of claim 1 when in bis-form.

3. A process for producing penicillin G or penicillin V comprising cyclisation of PCV or POCV respectively using a cyclase having isopenicillin-N-synthetase activity derived from a microorganism of a species of the genus Cephalosporium or Penicillium.

4. A process according to claim 3 in which the species is Cephalosporium acremonium (=Acremonium chrysogenum) or Penicillium chrysogenum.

5. A process for producing penicillin G comprising cyclisation of PCV using a cyclase having isopenicillin-N-synthetase activity derived from a microorganism of the species of the genus Streptomyces.

6. A process according to claim 5 in which the species is Streptomyces clavuligerus.

7. A process according to any of claims 3 to 6, in which the cyclase is provided as a cell-free extract of the microorganism.

8. A process according to any of claims 3 to 7, in which the PCV or POCV is formed in situ by reduction of the disulphide dimer.

**Revendications**

1. Un hétéropeptide arylacyl-cystéinyl-valine choisi parmi le phénylacétyl-L-cystéinyl-D-valine ("PCV") et le phénoxyacétyl-L-cystéinyl-D-valine ("POVC").

2. L'hétéropeptide de la revendication 1 lorsqu'il est dans la forme bis.

3. Un procédé pour la production de pénicilline G ou pénicilline V comprenant la cyclisation de respectivement PCV ou POCV en utilisant une cyclase qui a une activité d'isopénicilline-N-synthétase dérivée d'un microorganisme d'une espèce du genre Cephalosporium ou Penicillium.

4. Un procédé suivant la revendication 3 dans lequel l'espèce est le Cephalosporium acremonium - (*Acremonium chrysogenum) ou Penicillium chrysogenum.

5. Un procédé pour la production de pénicilline G comprenant la cyclisation de la PCV en utilisant une cyclase qui a une activité d'isopénicilline-N-synthétase dérivée d'un microorganisme de l'espèce du genre Streptomyces.

6. Un procédé suivant la revendication 5 dans lequel l'espèce est le Streptomyces clavuligerus.

7. Un procédé suivant l'une quelconque des revendications 3 à 6, dans lequel la cyclase est fournie sous la forme d'un extrait du microorganisme libéré des cellules.

8. Un procédé selon l'une quelconque des revendications 3 à 7, dans lequel la PCV ou la POCV est formé in situ par la réduction du dimère de di-sulfure.

**Patentansprüche**

1. Ein Arylacyl-Cysteinyl-Valin Heteropeptid, ausgewählt aus Phenylacetyl-L-Cysteinyl-D-Valin ("PCV") und Phenoxyacetyl-L-Cysteinyl-d-Valin ("POVC").

2. Das Heteropeptid nach Anspruch 1 in bis-Form.

3. Verfahren für die Herstellung von Penizillin G oder Penizillin V, umfassend die Zyklisierung von PCV respektive POCV unter Verwendung einer Zyklase, wobei die Isopenizillin-N-Synthetase-Wirkung von einem Mikroorganismus von einer Spezies der Gattung Cephalosporium oder Penicillium abgeleitet wird.

4. Verfahren nach Anspruch 3, in dem die Spezies Cephalosporium acremonium (=Acremonium chrysogenum) oder Penicillium chrysogenum ist.

5. Verfahren für die Herstellung von Penizillin G, umfassend die Zyklisierung von PCV unter Verwendung einer Zyklase, wobei die Isopenicillin-N-Synthetase-Wirkung von einem Mikroorganismus der Spezies der Gattung Streptomyces abgeleitet wird.

6. Verfahren nach Anspruch 5, in dem die Spezies Streptomyces clavuligerus ist.

7. Verfahren nach Anspruch 3 bis 6, in dem die Zyklase als zellfreier Extrakt des Mikroorganismus vorhanden ist.

8. Verfahren nach Anspruch 3 bis 7, in dem das PCV oder POCV in situ durch Reduzierung des Disulfid-Dimeres gebildet wird.

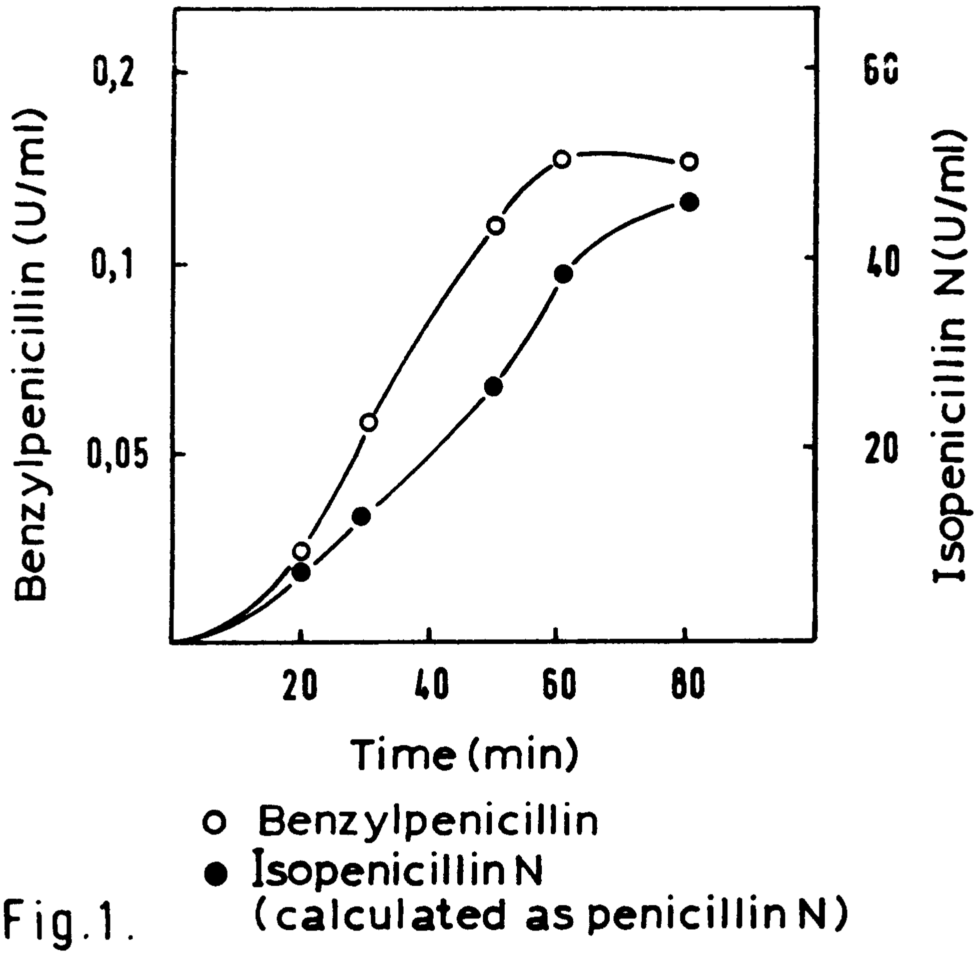
0,2
0,1
0,05
Benzylpenicillin (U/ml)
60
40
20
Isopenicillin N(U/ml)
20
40
60
80
Time (min)
o Benzylpenicillin
● Isopenicillin N
(calculated as penicillin N)

Fig.1.

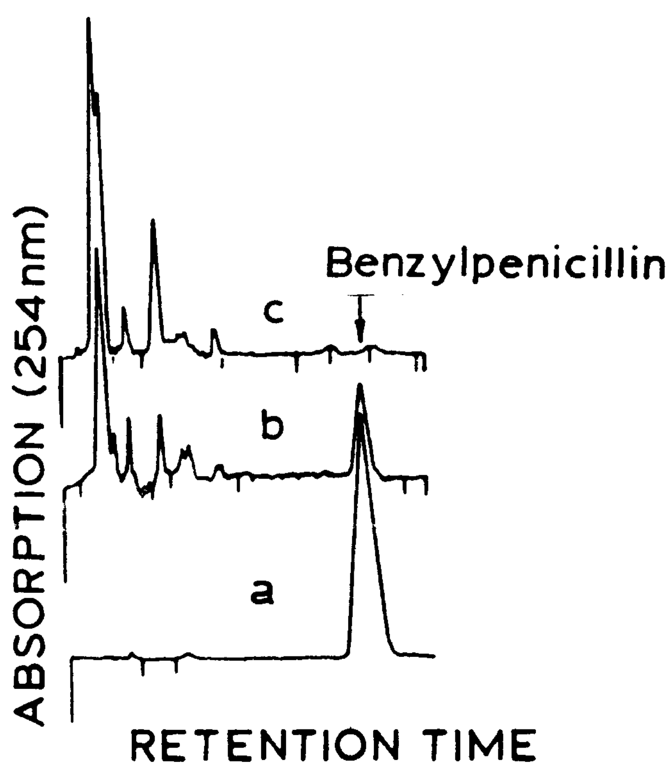
Benzylpenicillin
c
b
a
ABSORPTION (254nm)
RETENTION TIME

Fig.2.